# EUROPÄISCHE PATENTSCHRIFT

(11) **EP 2 736 408 B1**
(45) Veröffentlichungstag und Bekanntmachung des Hinweises auf die Patenterteilung: **16.08.2023**
(21) Anmeldenummer: 12732845.8
(22) Anmeldetag: 09.07.2012
(51) Int. Cl.: A61B 5/00, A61B 5/252

(54) **SAUGEINHEIT FÜR EIN EKG-GERÄT**
SUCTION UNIT FOR AN ECG APPLIANCE
UNITÉ D'ASPIRATION POUR UN APPAREIL D'ECC

(30) Priorität: 28.07.2011 CH 12612011
(43) Veröffentlichungstag der Anmeldung: 04.06.2014
(73) Patentinhaber: mbnet ag, 8105 Regensdorf (CH)
(72) Erfinder: BUCHER, Manuel, CH-8105 Watt- Regensdorf (CH)
(74) Vertreter: Clerc, Natalia
(86) Internationale Anmeldenummer: PCT/EP2012/063374
(87) Internationale Veröffentlichungsnummer: WO 2013/013962

(56) Entgegenhaltungen:
- WO-A2-2006/011144
- DE-A1- 2 837 279
- DE-U1- 20 311 343
- DE-U1- 29 820 332
- DE-U1-202005 012 455
- US-A1- 2006 161 068

## Beschreibung

### TECHNISCHES GEBIET

Die vorliegende Erfindung betrifft eine Saugeinheit für ein EKG-Gerät gemäss Oberbegriff des Patentanspruchs 1 sowie eine Haltevorrichtung gemäss Oberbegriff des Patentanspruchs 15.

### STAND DER TECHNIK

Elektrokardiogramm(EKG)-Geräte weisen eine Datenerfassungs- und Auswerteeinheit und damit verbundene Elektrodenleitungen mit Elektroden auf. Die Elektroden werden entweder auf die Haut des Patienten aufgeklebt oder mittels Unterdruck fixiert. Bei Verwendung von Unterdruck ist eine Sauganlage mit einer Vakuumpumpe notwendig.

EKG-Geräte mit Klebeelektroden lassen sich relativ klein und tragbar ausbilden. Beispiele hierfür sind aus DE 102 47 435, US 6 907 283, US 6 871 089 und US 7 361 188 bekannt.

EKG-Geräte mit Vakuumelektroden hingegen sind üblicherweise als fahrbare Standgeräte ausgeführt. Die Sauganlage umfasst die genannte Vakuumpumpe, eine Verbindung zur Datenerfassungs- und Auswerteeinheit, einen Mess- und Vakuumschlauch zu einer Verteilervorrichtung, die genannte Verteilervorrichtung sowie mehrere von dieser Verteilervorrichtung wegführende Elektrodenschläuche mit daran angeordneten Elektroden. Die Elektrodenschläuche beinhalten üblicherweise sowohl die elektrischen Messleitungen wie auch ein Lumen zum Anlegen des Unterdrucks an die Elektrode. Damit kein Kabel- bzw. Schlauchgewirr entsteht, ist üblicherweise ein am Fahrgestell des Standgeräts befestigter Halter mit einem Auslegearm vorhanden, an welchem die Verteilervorrichtung fixiert ist. Dieser Halter wird so nahe wie möglich an den Patienten herangeführt. Dies ist zwar bei liegenden Patienten durchführbar, jedoch beim Erfassen von Bewegungs-EKG's relativ schwierig. Die Elektrodenleitungen sind relativ lang und somit aufwendig in ihrer Handhabung. Sie stören auch den Patienten in seiner Bewegungsfreiheit. Die langen Elektrodenleitungen mit integriertem Sauglumen haben des Weiteren den Nachteil, dass die Messsignale störungsanfällig sind. Zudem besteht die Gefahr, dass die langen Leitungen beschädigt werden, z.B. dass sie eingeklemmt oder gar vom Fahrgestell überfahren werden. Ein weiterer Nachteil ist, dass die gesamte Anlage relativ viel Platz benötigt.

In DE 203 11 343 wird zwar vorgeschlagen, eine Auswerteschaltung bereits in der Verteilervorrichtung anzubringen. Es wird jedoch nach wie vor ein Standgerät mit den bekannten Nachteilen eingesetzt.

US 7 054 677 schlägt vor, die Sauganlage möglichst leicht auszubilden und versieht sie mit einem Traggriff. Die Schläuche weisen jedoch trotzdem eine relativ grosse Länge auf.

DE 20 2005 012 455 offenbart ein EKG-Gerät mit einer Sauganlage und einer Verarbeitungs- und Datenübertragungseinheit, wobei die Messsignale von den Elektroden über Schläuche der Sauganlage zur einer Sendeeinheit geleitet werden und von dort drahtlos an eine Empfangseinheit übermittelt werden. Auch hier sind relativ lange Schläuche von der Sauganlage zum Patienten notwendig.

WO 2006/011144 beschreibt ein Handschuh mit mehreren Saugelektroden und ein EKG Gerät mit integrierter Saugpumpe, welche an einem Arm befestigt werden kann. Vom Gerät führt eine Leitung zum Handschuh.

US 2006/0161068 zeigt ein EKG-System mit Mehrschichtschläuchen, welche ein Sauglumen und ein im Sauglumen verlaufendes Elektrodenkabel aufweist.

### DARSTELLUNG DER ERFINDUNG

Es ist eine Aufgabe der Erfindung, das Erfassen eines EKG's auch bei sich bewegenden Patienten zu erleichtern.

Diese Aufgabe löst eine Saugeinheit für ein EKG-Gerät mit den Merkmalen des Patentanspruchs 1.

Die erfindungsgemässe Saugeinheit weist ein Gehäuse auf, in welchem eine Vakuumpumpe angeordnet ist, wobei das Gehäuse eine Schnittstelle zur Übermittlung von Messsignalen an eine Datenempfangseinheit des EKG-Geräts aufweist. Das Gehäuse weist mehrere Anschlüsse für Elektrodenschläuche mit je mindestens einer Messleitung und je einer Saugleitung sowie mit je einer vakuumbeaufschlagbaren Vakuumelektrode auf.

Das Gehäuse aller Ausführungsformen ist vorzugsweise tragbar ausgebildet oder es ist an einem Auslegearm eines Halters befestigbar.

In einer Ausführungsform beträgt die Länge der Verbindung zwischen dem mindestens einen Anschluss des Gehäuses und den einzelnen Vakuumelektroden maximal circa 80 cm, vorzugsweise 40 bis 50 cm.

Insbesondere bei der tragbaren Ausführungsform der Saugeinheit sind diese kurzen Leitungen vorteilhaft.

In einer Ausführungsform ist eine Haltevorrichtung mit einem Auslegearm zur Verwendung mit einem EKG-Gerät vorhanden, wobei im oder am Auslegearm eine Saugeinheit für das EKG-Gerät angeordnet ist, wobei die Saugeinheit ein Gehäuse aufweist, in welchem eine Vakuumpumpe angeordnet ist und wobei das Gehäuse eine Schnittstelle zur Übermittlung von Messsignalen an eine Datenempfangseinheit des EKG-Geräts aufweist, wobei das Gehäuse mehrere Anschlüsse für Elektrodenleitungen mit je mindestens einer Messleitung und je einer Saugleitung sowie mit je einer vakuumbeaufschlagbaren Vakuumelektrode aufweist. In dieser Ausführungsform lassen sich je nach Art und Platzierung des Auslegearms auch längere Leitungen verwenden.

Die erfindungsgemässe Saugeinheit ist als separates Gerät ausgebildet und kann vom Patienten am Körper getragen oder im Bereich des Patienten befestigt werden. Es kann zum Beispiel am Patientenbett, an einem Laufband oder einem Testfahrrad fixiert werden. Hierfür sind vorzugsweise entsprechende Befestigungsmittel am Gehäuse der Saugeinheit angeordnet.

Es ist nun möglich, relativ kurze Elektrodenschläuche zwischen Vakuumpumpe und Vakuumelektroden zu verwenden.

Der Patient wird nicht mehr durch lange Schläuche in seiner Bewegung behindert. Die Handhabung der kurzen Schläuche ist für das medizinische Personal vereinfacht. Die Sauganlage kann optimal in Bezug auf den Patienten platziert werden. Insbesondere kann dadurch eine optimale Saugleistung und somit eine optimale Anlage der Elektroden auf dem Körper des Patienten erzielt werden. Der Platzbedarf bei der Verwendung der Saugeinheit, jedoch auch beim Transport zum Patienten und bei der Lagerung der Einheit bei Nichtgebrauch ist minimiert. Ein weiterer Vorteil ist, dass das Gerät im Vergleich zu den grossen Standgeräten einfacher verpackt und beispielsweise zu Servicezwecken kostengünstiger versandt werden kann.

Die Gefahr, dass die Schläuche beschädigt werden sowie dass Störungen im Messsignal auftreten, ist ebenfalls vermindert. Ein weiterer Vorteil ist, dass die gesamte EKG-Anlage dank der kurzen Schläuche nicht mehr so beeindruckend und furchteinflössend auf den Patienten wirkt. Der Patient ist ruhiger, und die Messsignale entsprechen dadurch eher einem normalen Verhalten des Patienten.

Die Schnittstelle zum EKG-Gerät ist vorzugsweise eine konventionelle elektrische Steckverbindung.

In einer bevorzugten Ausführungsform der Erfindung ist im Gehäuse eine Verteilervorrichtung vorgesehen mit einem Eingang und mehreren Ausgängen, wobei der Eingang mit einem Ausgang der Vakuumpumpe und je ein Ausgang der Verteilervorrichtung mit je einem der genannten Anschlüsse für die Elektrodenleitungen verbunden ist. Dadurch ist der Unterdruck, welcher von der Vakuumpumpe erzeugt wird, an die Elektrodenleitungen anlegbar.

In einer anderen Ausführungsform kann die Verteilervorrichtung am Gehäuse aufsteckbar oder über eine kurze Leitung mit diesem Gehäuse verbunden sein. In diesem Fall weist das Gehäuse vorzugsweise nur einen einzigen Anschluss auf.

Die Saugeinheit umfasst vorzugsweise ferner die genannten Elektrodenleitungen mit den damit verbundenen Vakuumelektroden. Diese Elektrodenleitungen sind vorzugsweise lösbar mit dem Gehäuse oder einer externen, der Saugeinheit benachbarten Verteilervorrichtung verbindbar. Sie können jedoch auch fest mit dem Gehäuse bzw. der gehäusenahen Verteilervorrichtung verbunden sein. Die Elektrodenleitungen einer Saugeinheit können alle gleich lang ausgebildet sein. Vorzugsweise sind sie unterschiedlich lang ausgebildet, wobei ihre Länge vorzugsweise in Abhängigkeit des Messortes am menschlichen Körper gewählt ist.

Vorzugsweise weisen die Elektrodenleitungen eine Länge von 40 bis 80 cm auf. Gute Resultate wurden für Elektrodenleitungen zur Messung im Bereich einer Brustwand erzielt, welche eine Länge von 40 bis 50 cm aufweisen, vorzugsweise von annähernd 40 cm. Elektrodenleitungen zur Messung im Bereich einer Peripherie des menschlichen Körpers, insbesondere im Bereich von Armen und Beinen, weisen vorzugsweise eine Länge von 50 bis 80 cm auf, vorzugsweise von annähernd 50 cm.

In der erfindungsgemässen Saugeinheit lassen sich bekannte Elektrodenleitungen mit integrierter Messleitung und Saugleitung verwenden. Vorzugsweise sind sie als Mehrfachleitungen ausgebildet, welche mindestens eine elektrische Signalleitung sowie eine Saugleitung beinhaltet. Vorzugsweise bildet die Saugleitung dabei ein zentral verlaufendes Lumen.

In einer bevorzugten erfindungsgemässen Ausführungsform ist die Elektrodenleitung jedoch aus einem Kunststoff gefertigt, insbesondere aus Silikon. Die Elektrodenleitung ist dabei vorzugsweise als Mehrschichtschlauch ausgebildet. In einer bevorzugten Ausführungsform umfasst die Elektrodenleitung mindestens vier, vorzugsweise genau vier Schichten. Eine äusserste erste Schicht ist dabei elektrisch isolierend, eine der ersten Schicht nachfolgende zweite Schicht elektrisch leitend, eine der zweiten Schicht nachfolgende dritte Schicht elektrisch isolierend und eine der dritten Schicht nachfolgende, innerste vierte Schicht elektrisch leitend ausgebildet. Die vierte Schicht bildet vorzugsweise den äusseren Mantel der Saugleitung. Diese Elektrodenleitung lässt sich einfach und kostengünstig herstellen und weist eine optimale Signalübermittlung auf. Vorteilhaft ist insbesondere, dass sie einen relativ kleinen Biegeradius der Elektrodenleitung erlaubt.

Weitere Ausführungsformen sind in den abhängigen Ansprüchen angegeben.

### KURZE BESCHREIBUNG DER ZEICHNUNGEN

Eine bevorzugte Ausführungsform der Erfindung ist im Folgenden anhand der Zeichnungen beschrieben, die lediglich zur Erläuterung dienen und nicht einschränkend auszulegen sind. In den Zeichnungen zeigen:
- Figur 1: eine Ansicht einer erfindungsgemässen Saugeinheit;
- Figur 2: eine perspektivische Darstellung der Saugeinheit gemäss Figur 1;
- Figur 3: eine Seitenansicht einer Elektrodenleitung der Saugeinheit gemäss Figur 1;
- Figur 4: eine perspektivische Ansicht der Elektrodenleitung gemäss Figur 3,
- Figur 5: einen Querschnitt durch die Elektrodenleitung gemäss Figur 3;
- Figur 6: eine Haltevorrichtung mit einer Aufnahme für die erfindungsgemässe Saugeinheit;
- Figur 7: einen Körpergurt mit einer Haltevorrichtung und Saugeinheit und
- Figur 8: einen Schultergurt mit Haltervorrichtung inklusive Saugeinheit.

### BESCHREIBUNG BEVORZUGTER AUSFÜHRUNGSFORMEN

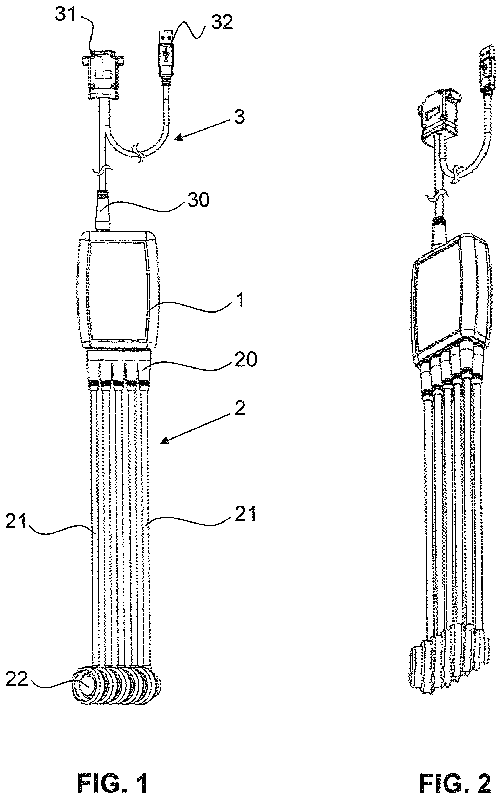

In den Figuren 1 und 2 ist eine erfindungsgemässe Saugeinheit dargestellt. Sie weist ein Gehäuse 1 mit einer darin angeordneten, hier nicht sichtbaren Vakuumpumpe auf. Vorzugsweise beinhaltet das Gehäuse 1 auch eine elektronische Steuerung zur Steuerung der Vakuumpumpe und gegebenenfalls Sensormittel zur Überwachung der Saugleistung der Vakuumpumpe bzw. der Saugeinheit.

Das Gehäuse 1 ist vorzugsweise quaderförmig ausgebildet und relativ flach. Es ist vorzugsweise mit hier nicht dargestellten Betätigungselementen zur Aktivierung und Betätigung der Vakuumpumpe bzw. zur manuellen Bedienung der elektronischen Steuerung versehen. Die Betätigungselemente sind bekannter Art, beispielsweise Schalter, Knöpfe oder Sensortastenfelder. Vorzugsweise ist die Anzahl der Betätigungselemente auf ein Minimum beschränkt, um eine einfache Bedienung zu gewährleisten.

Das Gehäuse 1 ist vorzugsweise mit Mitteln versehen, um es am Körper des Patienten oder benachbart zum Patienten, beispielsweise am Bett, einem Laufband oder einen Fahrrad, zu befestigen. Derartige Mittel können Haken, Klettverschlüsse oder Bänder sein. Auch lässt sich das Gehäuse 1 mit einem Band umwickeln und so an gewünschter Stelle befestigen.

Vorzugsweise ist das Gehäuse 1 auch mit einem Display versehen, um Angaben zur Pumpe und vorzugsweise auch über ein allenfalls angeschlossenes EKG-Gerät zu liefern. Des Weiteren können Angaben über die Elektrodenleitungen und die Elektroden angezeigt werden.

Die Vakuumpumpe ist vorzugsweise motorbetrieben. Im Gehäuse kann eine Batterie angeordnet sein oder die Vakuumpumpe kann an ein externes Stromversorgungsnetz oder an eine Batterie eines externen EKG-Geräts angeschlossen sein. Als Vakuummembranpumpe eignen sich bekannte Saugpumpen, beispielsweise Membranvakuumpumpen.

Das Gehäuse 1 verfügt über einen Anschluss zur Verbindung mit einem EKG-Gerät bekannter Art bzw. einer derartigen Datenerfassungs- und Auswerteeinheit. In den Figuren 1 und 2 ist ein Anschlussstecker 30 eines derartigen EKG-Geräts bereits in das Gehäuse 1 eingesteckt und eine entsprechende Daten- oder Verbindungsleitung 3, insbesondere bestehend aus elektrischen Leitungen, führen zu Steckern 31, 32. Diese Stecker 31, 32 lassen sich mit der entsprechenden, hier nicht dargestellten Datenerfassungs- und Auswerteeinheit bzw. mit einem zum EKG Gerät gehörigen Drucker oder Monitor verbinden.

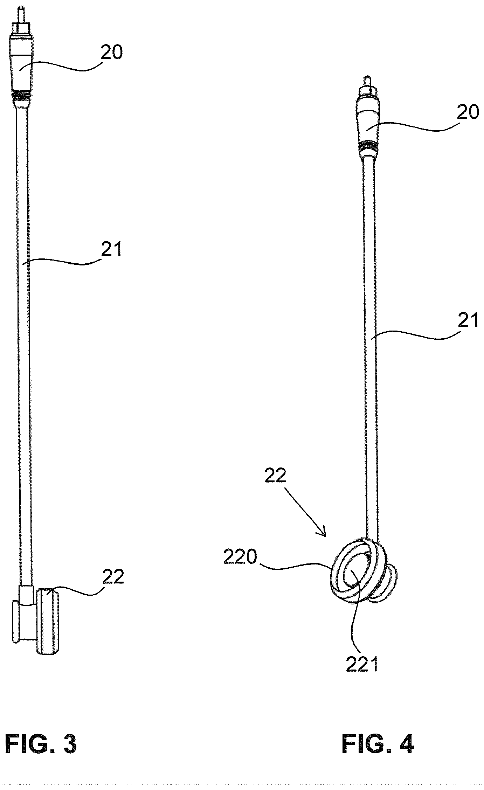

Auf der gegenüberliegenden schmalen Seite des Gehäuses 1 sind mehrere Anschlüsse für Elektrodenleitungen 2 vorhanden. Auch hier sind in den Figuren die entsprechenden Anschlussstecker 20 der Elektrodenleitungen bereits eingesteckt. Diese Stecker 20 sind jeweils je mit einem Schlauch 21 verbunden, wobei jeder Schlauch 21 mit seinem freien Ende in einer Messelektrode 22 endet. Dies ist in den Figuren 3 und 4 gut erkennbar.

Die Länge der Schläuche 21 ist hier im Vergleich zur Grösse des Gehäuses 1 nicht zwingend massstäblich dargestellt.

Wie in Figur 4 erkennbar ist, sind die Elektroden nach bekannter Art ausgebildet. Sie weisen einen umlaufenden Dichtungsring 220 auf, welcher auf die Haut des Patienten aufgelegt wird. Eine Elektrodenplatte 221 ist innerhalb des Dichtungsrings 220 angeordnet und kontaktiert die Haut zwecks Erstellung des EKG's. Der Dichtungsring 220 ist vorzugsweise aus einem weichen elastomeren Material, insbesondere Silikon gefertigt.

Die Elektrodenleitung 2 ist eine Mehrfachleitung. Sie weist mindestens eine, vorzugsweise zwei oder mehr elektrische Leitungen zur Verbindung mit der Elektrodenplatte 221 sowie eine hohle Saugleitung zur Verbindung mit dem durch den Dichtungsring 220 gebildeten Hohlraum auf.

Der Stecker 20 ist ein Stecker bekannter Art, welcher elektrische Verbindungen zu den elektrischen Leitungen sowie eine davon getrennte Verbindung zum Sauglumen ermöglicht. Der Anschluss im Gehäuse 1 der erfindungsgemässen Saugeinheit ist als entsprechendes Gegenstück ausgebildet. Derartige Steckverbindungen sind durch die Verteilervorrichtungen der bekannten EKG-Geräte offenbart.

Damit der von der Vakuumpumpe erzeugte Unterdruck an die Vielzahl der Anschlüsse des Gehäuses 1 übermittelt und somit an die Sauglumen und die Elektroden 221 angelegt werden kann, ist im Gehäuse vorzugsweise eine hier nicht dargestellte Verteilervorrichtung angeordnet, welche den Vakuumausgang der Vakuumpumpe mit den einzelnen Anschlüssen des Gehäuses 1 verbindet.

Die elektrische Verbindung zwischen den Steckern 20 der Elektrodenleitungen 2 bzw. den zugehörigen elektrodenseitigen Anschlüssen des Gehäuses 1 sowie dem EKG-gerätseitigen Anschluss des Gehäuses 1 und somit dem Stecker 30 der Verbindungsleitung 3 kann im Gerät ebenfalls über diese Verteilervorrichtung erfolgen oder getrennt von dieser verlaufen. Letzteres ist bevorzugt, da dadurch die Verteilervorrichtung einfacher und kleiner ausgebildet ist.

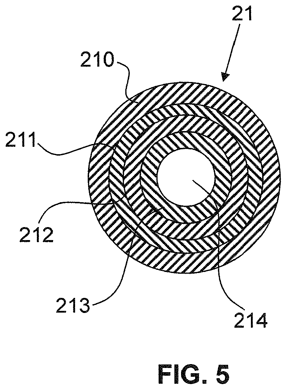

Als Elektrodenleitungen 2 lassen sich Leitungen bekannter Art verwenden. Vorzugsweise wird jedoch eine Mehrschichtleitung verwendet, welche mindestens, vorzugsweise genau vier Schichten aufweist. Diese Elektrodenleitung ist in Figur 5 dargestellt. Sie besteht vorzugsweise aus einem Kunststoff. Besonders geeignet sind Kunststoffe auf Silikonbasis. Eine erste äusserste Schicht 210 ist elektrisch isolierend ausgebildet. Eine zweite, der ersten Schicht 210 angrenzende Schicht 211 ist elektrisch leitend ausgebildet, eine dritte, der zweiten Schicht 211 angrenzende Schicht 212 ist wieder elektrisch isolierend ausgebildet und eine innerste vierte Schicht 213, welche wiederum an die dritte Schicht 212 angrenzt, ist elektrisch leitend ausgebildet. In der Mitte ist ein zentral verlaufendes Lumen 214 vorhanden, welches die Saugleitung bildet.

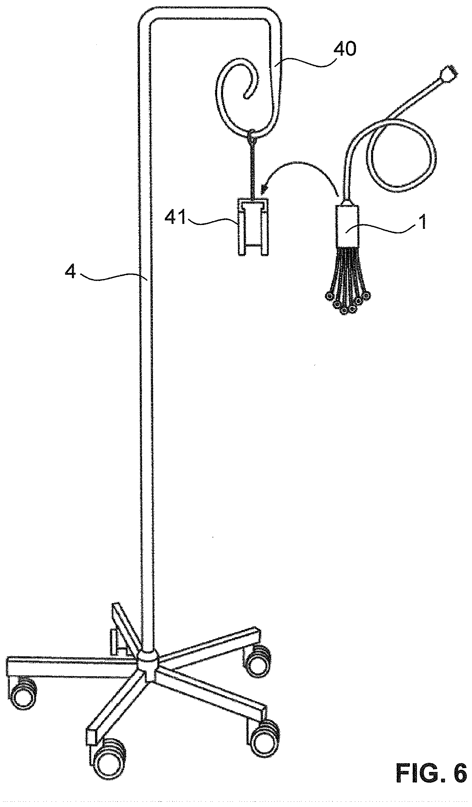

In Figur 6 ist eine weitere Ausführungsform der Erfindung dargestellt. Hier ist die Vakuumpumpe mit ihrem Gehäuse 1 in einem Auslegearm 40 eines Halters 4 angeordnet. Der Halter ist vorzugsweise ein Halter eines fahrbaren Standgeräts einer EKG-Vorrichtung oder ein Auslegearm, welches beispielsweise an einem stationären Trainingsgerät, wie beispielsweise ein Fahrrad oder ein Laufband, befestigt ist. Es kann sich aber auch um einen anderen fahrbaren oder stationären Halter handeln.

Der Auslegearm 40 weist eine Aufnahme 41 auf, in welchem das Gehäuse der Vakuumpumpe lösbar gehalten ist. Die Aufnahme 41 kann Bestandteil des Auslegearms sein, oder, wie hier dargestellt, lösbar mit diesem verbunden sein. Hier ist die Aufnahme 41 am Auslegearm 40 angehängt. Alternativ kann das Gehäuse 1 der Vakuumpumpe integraler Bestandteil des Auslegearms 40 und für den Benützer ohne Werkzeug nicht lösbar vom restlichen Auslegearm angeordnet sein.

Die Aufnahme 41 besteht vorzugsweise aus einem quaderförmigen Grundkörper mit einer ein- oder beidseitig offenen Nut, in welche das Gehäuse 1 eingeschoben werden kann. Die Aufnahme ist vorzugsweise aus Kunststoff oder Metall gefertigt.

Wird die Vakuumpumpe mit einem Auslegearm verwendet, so sind die Elektrodenleitungen und Schläuche zum Patienten vorzugsweise länger ausgebildet als im ersten Ausführungsbeispiel.

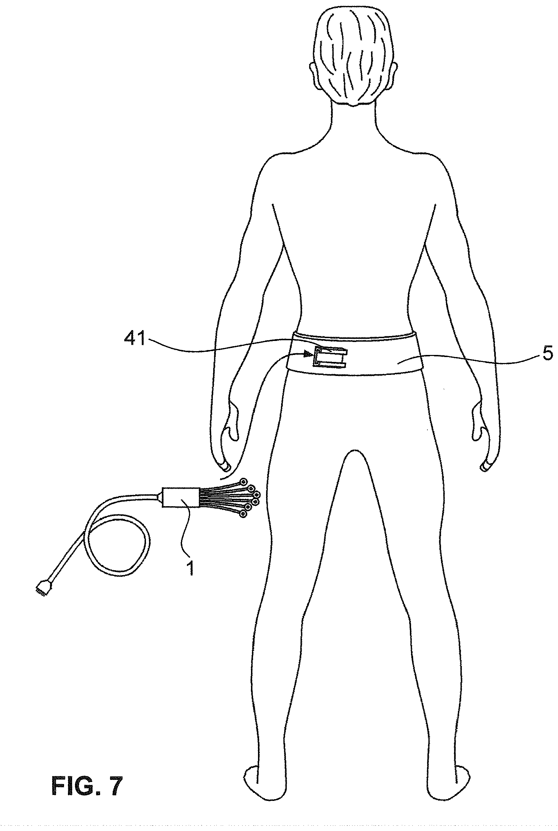

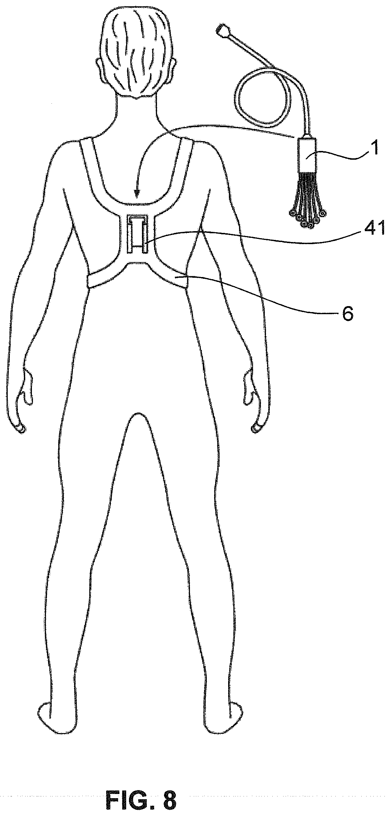

Figur 7 zeigt diese Haltevorrichtung 41 an einem Körpergurt 5 befestigt. Figur 8 zeigt die Verwendung mit einem Schultergurt 6.

Die erfindungsgemässe Saugeinheit zur Verwendung in einer EKG-Anlage ist klein und ermöglicht den Einsatz von relativ kurzen Elektrodenleitungen. Die gesamte EKG-Anlage wird dadurch mobiler, und sie ist vielfältiger einsetzbar.

### BEZUGSZEICHENLISTE

| | | | |
|---|---|---|---|
| 1 | Gehäuse | | |
| | | 3 | Verbindungsleitung |
| 2 | Elektrodenleitung | 30 | Anschlussstecker |
| 20 | Anschlussstecker | 31 | Verbindungsstecker |
| 21 | Schlauch | 32 | Verbindungsstecker |
| 210 | erste Schicht | | |
| 211 | zweite Schicht | 4 | Halter |
| 212 | dritte Schicht | 40 | Auslegearm |
| 213 | vierte Schicht | | |
| 214 | zentrales Lumen | 5 | Körpergurt |
| 22 | Elektrode | | |
| 220 | Dichtungsring | 6 | Schultergurt |
| 221 | Elektrodenplatte | | |

## Patentansprüche

1. Saugeinheit für ein EKG-Gerät, wobei die Saugeinheit ein Gehäuse (1) aufweist, in welchem eine Vakuumpumpe angeordnet ist und wobei das Gehäuse (1) eine Schnittstelle zur Übermittlung (3) von Messsignalen an eine Datenempfangseinheit des EKG-Geräts aufweist, **dadurch gekennzeichnet, dass** die Saugeinheit als separates Gerät ausgebildet ist, dass das Gehäuse (1) mehrere Anschlüsse für Elektrodenleitungen (2) mit je mindestens einer Messleitung (211, 213) und je einer Saugleitung (214) sowie mit je einer vakuumbeaufschlagbaren Vakuumelektrode (22) aufweist und dass das Gehäuse einen EKG-gerätseitigen Anschluss aufweist für einen Anschlussstecker (30) einer Verbindungsleitung (3) des EKG-Geräts.

2. Saugeinheit nach Anspruch 1, wobei das Gehäuse (1) tragbar ausgebildet ist oder an einem Auslegearm befestigbar ist.

3. Saugeinheit nach einem der Ansprüche 1 oder 2, wobei im Gehäuse (1) eine Verteilervorrichtung vorgesehen ist mit einem Eingang und mehreren Ausgängen, wobei der Eingang mit einem Ausgang der Vakuumpumpe und je ein Ausgang der Verteilervorrichtung mit je einem der genannten Anschlüsse für die Elektrodenleitungen (2) verbunden ist, wodurch Unterdruck, welcher von der Vakuumpumpe erzeugt wird, an die Saugleitungen der Elektrodenleitungen (2) anlegbar ist.

4. Saugeinheit nach einem der Ansprüche 1 bis 3, wobei sie ferner die Elektrodenleitungen (2) mit den damit verbundenen Vakuumelektroden (22) umfasst.

5. Saugeinheit nach Anspruch 4, wobei die Elektrodenleitungen (2) eine Länge von 40 bis 80 cm aufweisen.

6. Saugeinheit nach einem der Ansprüche 4 oder 5, wobei mindestens ein Teil der Elektrodenleitungen (2) zur Messung im Bereich einer Brustwand ausgebildet ist und eine Länge von 40 bis 50 cm aufweisen, vorzugsweise von annähernd 40 cm.

7. Saugeinheit nach einem der Ansprüche 4 bis 6, wobei mindestens ein Teil der Elektrodenleitungen (2) zur Messung im Bereich einer Peripherie des menschlichen Körpers, insbesondere im Bereich von Armen und Beinen, ausgebildet ist und eine Länge von 50 bis 80 cm aufweisen, vorzugsweise von annähernd 50 cm.

8. Saugeinheit nach einem der Ansprüche 1 bis 7, wobei jede Elektrodenleitung (2) eine Mehrfachleitung ist, welche mindestens eine elektrische Signalleitung (211, 213) sowie eine Saugleitung (214) beinhaltet.

9. Saugeinheit nach Anspruch 8, wobei die Saugleitung (214) ein zentrales Lumen in der Elektrodenleitung (2) ist.

10. Saugeinheit nach einem der Ansprüche 8 oder 9, wobei die Elektrodenleitung (2) aus Kunststoff, insbesondere einem auf Silikon basierenden Kunststoff, gefertigt ist.

11. Saugeinheit nach einem der Ansprüche 8 bis 10, wobei die Elektrodenleitung (2) ein Mehrschichtschlauch ist.

12. Saugeinheit nach Anspruch 11, wobei die Elektrodenleitung (2) vier Schichten umfasst, wobei eine äusserste erste Schicht (210) elektrisch isolierend, eine der ersten Schicht (210) nachfolgende zweite Schicht (211) elektrisch leitend, eine der zweiten Schicht (211) nachfolgende dritte Schicht (212) elektrisch isolierend und eine der dritten Schicht (212) nachfolgende, innerste vierte Schicht (213) elektrisch leitend ausgebildet ist.

13. Saugeinheit nach Anspruch 12, wobei die Elektrodenleitung (2) genau vier Schichten umfasst und die vierte Schicht (213) den äusseren Mantel der Saugleitung (214) bildet.

14. Saugeinheit nach einem der Ansprüche 1 bis 13, wobei das Gehäuse (1) ein Mittel zur Befestigung des Gehäuses am menschlichen Körper aufweist.

15. Haltevorrichtung mit einem Auslegearm zur Verwendung mit einem EKG-Gerät, wobei im oder am Auslegearm eine Saugeinheit für das EKG-Gerät angeordnet ist, wobei die Saugeinheit gemäss einem der Ansprüche 1 bis 14 ist.

## Claims

1. Suction unit for an ECG apparatus, the suction unit comprising a housing (1) in which a vacuum pump is arranged and the housing (1) comprising an interface for transmitting (3) measurement signals to a data receiver unit of the ECG apparatus, **characterized in that** the suction unit is designed as a separate apparatus, **in that** the housing (1) comprises a plurality of connectors for electrode cables (2), each with at least one measuring line (211, 213) and one suction line (214) and one vacuum electrode (22) to which the vacuum can be applied, and **in that** the housing comprises an ECG apparatus-side connector for a connection plug (30) of a connecting line (3) of the ECG apparatus.

2. Suction unit according to Claim 1, wherein the housing (1) has a portable design or is attachable to an extension arm.

3. Suction unit according to either of Claims 1 and 2, wherein a distribution device with one input and a plurality of outputs is provided in the housing (1), the input being connected to an output of the vacuum pump and a respective output of the distribution device being connected to a respective aforementioned connector for the electrode cables (2), as a result of which negative pressure produced by the vacuum pump is able to be applied to the suction lines of the electrode cables (2).

4. Suction unit according to any one of Claims 1 to 3, further comprising the electrode cables (2) with the vacuum electrodes (22) connected therewith.

5. Suction unit according to Claim 4, wherein the electrode cables (2) have a length of 40 to 80 cm.

6. Suction unit according to either of Claims 4 and 5, wherein at least some of the electrode cables (2) are designed to measure in the region of a thoracic wall and have a length of 40 to 50 cm, preferably approximately 40 cm.

7. Suction unit according to any one of Claims 4 to 6, wherein at least some of the electrode cables (2) are designed to measure in the region of a periphery of the human body, in particular in the region of arms and legs, and have a length of 50 to 80 cm, preferably approximately 50 cm.

8. Suction unit according to any one of Claims 1 to 7, wherein each electrode cable (2) is a multi-line containing at least one electrical signal line (211, 213) and a suction line (214).

9. Suction unit according to Claim 8, wherein the suction line (214) is a central lumen in the electrode cable (2).

10. Suction unit according to either of Claims 8 and 9, wherein the electrode cable (2) is manufactured from a plastic, in particular a silicone-based plastic.

11. Suction unit according to any one of Claims 8 to 10, wherein the electrode cable (2) is a multilayer tubing.

12. Suction unit according to Claim 11, wherein the electrode cable (2) comprises four layers, with an outermost first layer (210) being designed to be electrically insulating, a second layer (211) following the first layer (210) being designed to be electrically conductive, a third layer (212) following the second layer (211) being designed to be electrically insulating and an innermost fourth layer (213) following the third layer (212) being designed to be electrically conductive.

13. Suction unit according to Claim 12, wherein the electrode cable (2) comprises exactly four layers and the fourth layer (213) forms the outer jacket of the suction line (214).

14. Suction unit according to any one of Claims 1 to 13, wherein the housing (1) comprises a means for fastening the housing to the human body.

15. Holding equipment having an extension arm for use with an ECG apparatus, a suction unit for the ECG apparatus being arranged in or on the extension arm, the suction unit being in accordance with any one of Claims 1 to 14.

## Revendications

1. Unité d'aspiration destinée à un appareil ECG, l'unité d'aspiration comportant un boîtier (1) dans lequel est disposée une pompe à vide et le boîtier (1) comportant une interface destinée à transmettre (3) des signaux de mesure à une unité de réception de données de l'appareil ECG, **caractérisée en ce que** l'unité d'aspiration est conçue comme un appareil séparé, **en ce que** le boîtier (1) comporte plusieurs connecteurs destinés à des lignes d'électrode (2) pourvues chacune d'au moins une ligne de mesure (211, 213) et d'une ligne d'aspiration (214) et pourvue chacune d'une électrode à vide (22) qui peut être soumise à un vide et **en ce que** le boîtier comporte un connecteur côté appareil ECG destiné à une prise de connexion (30) d'une ligne de liaison (3) de l'appareil ECG.

2. Unité d'aspiration selon la revendication 1, le boîtier (1) étant conçu pour être portable ou pouvant être fixé à un bras d'extension.

3. Unité d'aspiration selon l'une des revendications 1 ou 2, un dispositif distributeur étant prévu dans le boîtier (1) et comportant une entrée et plusieurs sorties, l'entrée étant reliée à une sortie de la pompe à vide et les sorties du dispositif distributeur étant reliées respectivement à l'un desdits connecteurs destinés aux lignes d'électrode (2), ce qui permet d'appliquer une pression négative, qui est générée par la pompe à vide, aux lignes d'aspiration des lignes d'électrode (2).

4. Unité d'aspiration selon l'une des revendications 1 à 3, laquelle comprend en outre les lignes d'électrode (2) auxquelles les électrodes à vide (22) sont reliées.

5. Unité d'aspiration selon la revendication 4, les lignes d'électrode (2) ayant une longueur de 40 à 80 cm.

6. Unité d'aspiration selon l'une des revendications 4 ou 5, au moins une partie des lignes d'électrode (2) étant conçue pour effectuer une mesure dans la région d'une paroi thoracique et ayant une longueur de 40 à 50 cm, de préférence d'environ 40 cm.

7. Unité d'aspiration selon l'une des revendications 4 à 6, au moins une partie des lignes d'électrode (2) étant conçue pour effectuer une mesure dans la région d'une périphérie du corps humain, en particulier dans la région des bras et des jambes, et ayant une longueur de 50 à 80 cm, de préférence d'environ 50 cm.

8. Unité d'aspiration selon l'une des revendications 1 à 7, chaque ligne d'électrode (2) étant une ligne multiple comprenant au moins une ligne de signal électrique (211, 213) et une ligne d'aspiration (214).

9. Unité d'aspiration selon la revendication 8, la ligne d'aspiration (214) étant une lumière centrale dans la ligne d'électrode (2).

10. Unité d'aspiration selon l'une des revendications 8 ou 9, la ligne d'électrode (2) étant en matière synthétique, en particulier une matière synthétique à base de silicone.

11. Unité d'aspiration selon l'une des revendications 8 à 10, la ligne d'électrode (2) étant un tuyau multicouche.

12. Unité d'aspiration selon la revendication 11, la ligne d'électrode (2) comprenant quatre couches, une première couche la plus à l'extérieur (210) étant électriquement isolante, une deuxième couche (211) faisant suite à la première couche (210) étant électriquement conductrice, une troisième couche (212) faisant suite à la deuxième couche (211) étant électriquement isolante et une quatrième couche la plus à l'intérieur (213) faisant suite à la troisième couche (212) étant électriquement conductrice.

13. Unité d'aspiration selon la revendication 12, la ligne d'électrode (2) comprenant exactement quatre couches et la quatrième couche (213) formant l'enveloppe extérieure de la ligne d'aspiration (214).

14. Unité d'aspiration selon l'une des revendications 1 à 13, le boîtier (1) comportant des moyens destinés à fixer le boîtier au corps humain.

15. Dispositif de retenue comprenant un bras d'extension destiné à être utilisé avec un appareil ECG, une unité d'aspiration destinée à l'appareil ECG étant disposée dans ou sur le bras d'extension, l'unité d'aspiration étant selon l'une des revendications 1 à 14.
